# EUROPEAN PATENT SPECIFICATION

(11) **EP 0 692 278 B1**
(45) Date of publication and mention of the grant of the patent: **11.10.2000**
(21) Application number: 95110934.7
(22) Date of filing: 12.07.1995
(51) Int. Cl.: A61M 39/06

(54) **Catheter hemostasis valve**
Katheterhämostatisches Ventil
Valve hémostatique pour cathéter

(30) Priority: 15.07.1994 US 275828
(43) Date of publication of application: 17.01.1996
(73) Proprietor: Cordis Corporation, Miami Lakes Florida 33014 (US)
(72) Inventor: Davila, Luis A., Cooper City, Florida 33330 (US); De La Mata, Carlo R., North Miami Beach, Florida 33160 (US)
(74) Representative: Kuhnen & Wacker

(56) References cited:
- EP-A- 0 369 314
- EP-A- 0 442 194
- US-A- 4 000 739
- US-A- 4 895 565

## Description

### BACKGROUND OF THE INVENTION

Catheters, or catheter sheath introducers, having hemostasis valves which are mounted in a housing on the end of a catheter are well known in the art. Examples of such devices are given in U.S. Patent Nos. 4,798,594, 4,895,565, 4,000,739 and 4,421,296. Such a catheter is used to facilitate the introduction of other catheters and guidewires into the vascular system of a patient, while minimizing injury to the patient at the access site. This is particularly used in situations where one or more catheters are inserted into and removed from the patient repeatedly, for example as in angioplasty. The presence of the catheter sheath introducer causes the trauma to the body to be limited to only one catheter entering at the body access site. All other catheters and guidewires pass through the catheter introducer, and thus are not traumatic to the body at the access site. EP 369 314 describes a catheter sheath introducer according to the first part of claim 1.

Catheter sheath introducers are known to carry a hemostasis valve which comprises a slit elastomeric partition or membrane carried in a housing. While the prior art shows many different designs of such membrane type hemostasis valves, there is still a desire for improvement in the performance of such valves with respect to their ability to seal against leakage of blood, as catheters and guidewires of varying diameters are passed through the valve. Also, it is important for the friction encountered as one advances a catheter or a guidewire through a catheter sheath introducer to be as low as possible. By this invention, improvements in both the sealing characteristics of the membrane valve of this invention and the frictional characteristics of the valve are achieved, to provide a partition valve for a catheter which exhibits improved characteristics over the prior art.

### SUMMARY OF THE INVENTION

In accordance with the present invention there is provided a catheter according to claim 1.

In accordance with another aspect of the present invention, the catheter described above preferably has a valve partition containing from about 5 to 20 weight percent of a lubricity enhancing additive such as bismuth oxychloride, polytetrafluoroethylene, titanium dioxide, graphite, polyethylene wax, polyvinylpyrrolidone, and combinations thereof.

### DESCRIPTION OF THE DRAWINGS

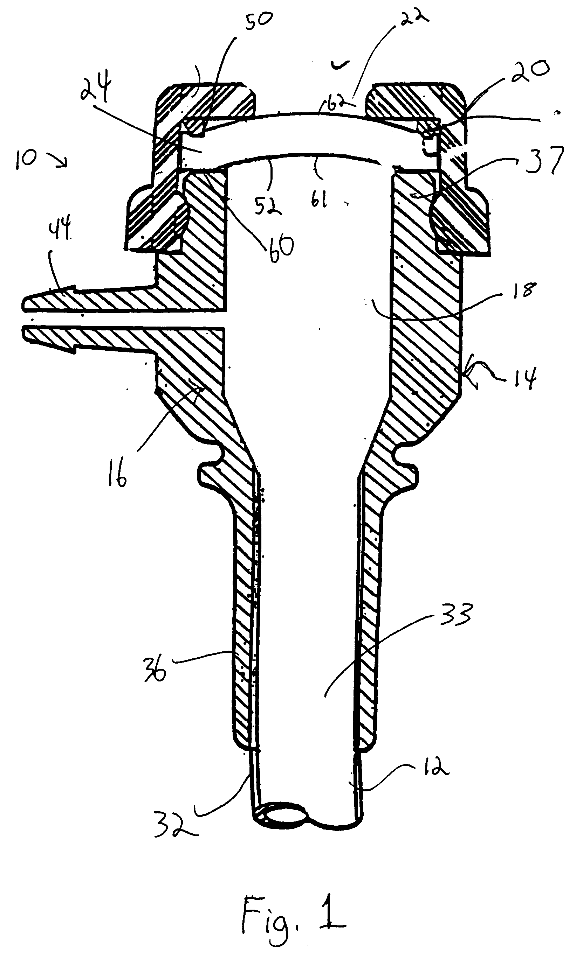

In the drawings, Fig. 1 is a fragmentary, longitudinal section view of a catheter introducer in accordance with the present invention.

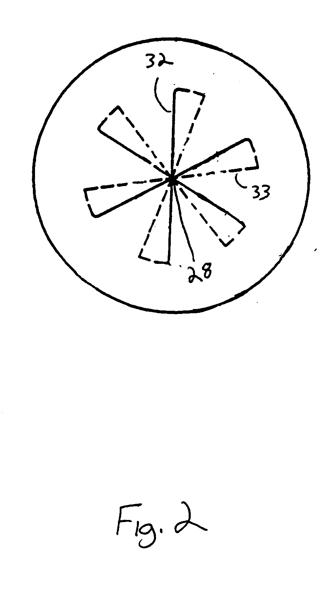

Fig. 2 is an enlarged plan view of the valve partition used in the catheter introducer of Fig. 1.

### DETAILED DESCRIPTION OF THE INVENTION

Referring to the drawings, Fig. 1 shows a portion of a catheter, or catheter sheath introducer, which is of conventional construction except as otherwise indicated herein. Catheter introducer 10 comprises a tubular catheter 12 having a distal end (not shown), a proximal end 32 and a lumen 33 extending therethrough. Catheter introducer 10 carries a hemostasis valve 14 on one end thereof. Hemostasis valve 14 comprises a housing 16, having a distal end 36, a proximal end 37 and a bore 18 extending therethrough. The distal end 36 of housing 16 is attached, by any suitable means known in the art, to the proximal end 32 of catheter 12. Bore 18 communicates with lumen 33 of catheter 12. Housing 16 has an end wall 60 at its proximal end 37. Housing 16 is closed by an end cap 20 being snap-fit and/or heat sealed into the disclosed position on inner body 16. End cap 20 has a central aperture 22. Catheter inner body 16 defines the typical side port 44 found in catheter introducers.

Hemostasis valve 14 includes a slit elastomeric valve partition 24, typically a circular disk, having opposing major surfaces 61 and 62. Valve partition 24 is sealed along its periphery between end wall 60 and end cap 20, as shown. Cap 20 has an aperture 22, wherein bore 18 of housing 16 and aperture 22 of cap 20 communicate with major surfaces 61 and 62 of valve partition 24 respectively.

Cap 10 includes a compression ring 50 abutting against the periphery of valve partition 24. Compression ring 50 substantially surrounds the end cap aperture 22 and the portion of housing bore 18 which is adjacent to partition 24. Compression ring 50 has a larger inner diameter than aperture 22 and bore portion 18. However, compression ring 50 is of no more than equal diameter to that of the partition 24. This is because ring 50 preferably engages the partition along its periphery so as to press the peripheral portion of the partition to a greater degree than the rest of the end cap 20 presses against the partition. The effect of this, when the inner diameter of ring 50 is greater than the diameter of bore portion 18, is to cause partition 24 to bow outwardly as shown in Fig. 1, and particularly to project outwardly to a degree into aperture 22. This outward bowing is facilitated by the space provided underneath end cap 20 by the presence of ring 50. The effect of such bowing enhances the sealing of the slit partition 24, particularly in the portion adjacent the inner surface 52 of partition 24. Adjustment of the bowing of partition 24 a can be made by variation of the dimensions of ring 50 and its respective diameter compared with the inner diameter of bore 18. The bowing phenomenon is achieved primarily when the inner diameter of ring 50 is greater than the diameter of bore portion 18.

The slits of valve partition 24 may be of any known design, but preferably comprises a plurality of slits 32 (Fig. 2) extending out from the center 28 and extending through partition 24 at an angle. Slits 32 are rotated in helical manner as they extend through the partition and exit the other side of the partition as noted by dotted lines 33. The radial slits rotate circumferentially by about 5° to 60° as they extend through the valve partition from one face to the other. This helical structure is similar to that disclosed in Hillstead U.S. Patent Nos. 4,798,594 and 4,895,565. While the specific slit pattern shown comprises six slits in a cross pattern that extend first in a helical and then a spiral pattern, it is to be understood that other helical and spiral slit arrays may also be used. Furthermore, any such partition known to those skilled in the art can be used.

Further in accordance with this invention, the slit partition 24 is preferably formed from silicone rubber, which preferably may have an elongation to break of at least 900, and typically no more than 1500 percent, as measured by the usual and conventional ASTM D412. The typical prior art slit partition hemostasis valves have a maximum elongation of about 750 percent as measured in similar manner. It has been found that the higher elongations used help to provide improved sealing characteristics over partitions of lower elongation.

Also it is preferred for the elastomeric material to have a tensile strength of at least 11.5 megapascals up to generally a practical maximum of about 15 megapascals as measured by the same ASTM D412. The typical prior art silicone partition valves have a tensile strength of about 10.9 megapascals, significantly below the tensile strength of the preferred elastomer materials used herein. It is also preferred for the elastomeric materials used herein to have a Shore A durometer of about 20 to 50, although higher durometers may be used if desired. Specifically, silicone elastomer materials which are preferred for use herein are available from the Dow Corning Corporation of Midland, Michigan under the name Silastic Q7-47-20, 35, or 50 medical grade ETR elastomer. The later two digit numbers refer to the Shore A durometers of the material. A preferred tear strength of the elastomers used is at least 26,79 kg/cm (150 pounds/inch) as determined by ASTM D624, Die B.

In accordance with this invention, the slit partition which is preferably made of an elastomer such as the ones described above, and also contains an effective amount of a lubricity enhancing additive which is a finely divided material selected from the group consisting of bismuth oxychloride, polytetrafluorethylene, titanium dioxide, graphite, polyethylene wax, polyvinylpyrrolidone, and combinations thereof. The lubricity enhancing additives are typically milled into the uncured elastomer, so as to be substantially uniformly distributed throughout the elastomer material.

Preferably, graphite is present in a concentration of about 5-15 weight percent. The other specifically mentioned lubricity enhancing additives may be present in a total amount of about 5 to 20 weight percent in the elastomer formulation.

The lubricity enhancing additives provided by this invention not only reduce the friction which is encountered when a catheter or guidewire is advanced through the slit partition and the catheter which carries it, but also, the lubricity enhancing additives serve to prevent the leaflets of elastomer material defined by the slits in the partition valve from sticking to each other with high force. Especially in the situation where the preferred elastomers of high elongation are used as the valve partition, it is possible for the slits which are cut in partitions made of the unmodified elastomer to actually reseal because of the highly tacky characteristic of most silicone elastomers of high elongation. The presence of one of more the additives of this invention can reduce or eliminate this phenomenon, while also reducing frictional forces as a catheter or guidewire is advanced through said valve partition.

It is generally preferred for the additives to be present in a concentration of about 7 to 13 weight percent. By way of specific embodiment, formulations of the above described silicone elastomer have been prepared containing 10 weight percent of, respectively, bismuth oxychloride, PTFE, titanium dioxide, polyethylene wax, and polyvinylpyrrolidone. A similar formulation of such a silicone elastomer has been prepared containing 7.5 weight percent of graphite. The specific Shore A durometers of the silicone rubber silastic ETR Q7-47 formulations tested were 20, 35, and 50. Each of these formulations may respectively contain the above specific amounts of 10 or 7.5 percent of a lubricity enhancing additive as stated, to achieve the desirable results of this invention when formulated into a partition valve and mounted into a catheter sheath introducer.

The above has been offered for illustrative purposes only, and is not intended to limit the scope of the invention of this application, which is as defined in the claims below.

## Claims

1. A catheter sheath introducer (10) having a tubular catheter (12) comprising a distal end, a proximal end (32) and a lumen (33) extending therethrough, the catheter carries a hemostasis valve (14) on its proximal end (32), the hemostasis valve (14) comprises a housing (16) whose distal end (36) is attached to the proximal end (32) of the catheter (12), the housing (16) has a bore (18) extending therethrough which is in communication with the lumen (33) of the catheter (12), the housing (16) has an end wall (60) at its proximal end (37) surrounding the bore (18), the hemostasis valve (14) further includes a slit elastic valve partition (24) with opposing major surfaces (61, 62), the valve partition (24) is peripherally sealed between the end wall (60) and a cap (20), the cap (20) has an aperture (22) extending therethrough, the bore (18) of the housing (16) and the aperture (22) of the cap (20) each contact an opposed major surface (61, 62) of the valve partition (24), the hemostasis valve (14) further comprising
a compression ring (50) disposed on the cap (20) and abutting against the periphery of the valve partition (24),
characterised in that
the compression ring (50) has a larger inner diameter than the aperture (22) and housing bore (18) and of no more than equal diameter to that of the valve partition (24), the compression ring (50) presses the periphery of the partition (24) against the housing (16), causing the center of the valve partition (24) to bow outwardly towards the aperture (22), and
said valve partition (24) is formed by a circular disk, so that said bowing outwardly enhances the sealing of the slit partition in a portion adjacent the major surface (61) contacting said end wall (60).

2. The catheter sheath introducer of claim 1 wherein the center of the valve partition bows outwardly so as to project into the aperture (22) of the cap (20).

3. The catheter sheath introducer according to any of the previous claims wherein the valve partition (24) is made of an elastomer.

4. The catheter sheath introducer according to any of the previous claims in which the valve partition (24) comprises from about 5 to 20 weight percent of a lubricity enhancing additive selected from the group consisting of bismuth oxychloride, polytetrafluoroethylene, titanium dioxide, graphite, polyethylene wax, polyvinylpyrrolidone, and combinations thereof.

5. The catheter sheath introducer according to any of the previous claims in which the valve partition (24) comprises from about 5 to 15 weight percent of graphite.

6. The catheter sheath introducer according to any of the previous claims in which the valve partition (24) defines from 4 to 6 of the radial slits (32).

7. The catheter sheath introducer according to claim 6 where the slits (32) extend through the valve partition (24) at an angle from one major surface to another.

8. The catheter sheath introducer according to claim 7 wherein the slits (32) extend through the valve partition (24) an an angle from 5° to 60°.

9. The catheter sheath introducer according to any of the previous claims, wherein a space is provided underneath said cap (20) by the presence of said ring (50) in order to facilitate said outward bowing.

## Patentansprüche

1. Katheter-Mantelintubator (10) mit einem rohrförmigen Katheter (12), der ein distales Ende, ein proximales Ende (32) und ein sich hindurch erstreckendes Lumen (33) enthält, wobei der Katheter ein hämostatisches Ventil (14) an seinem proximalen Ende (32) trägt, wobei das hämostatische Ventil (14) ein Gehäuse (16) aufweist, dessen distales Ende (36) an das proximale Ende (32) des Katheters (12) angefügt ist, wobei das Gehäuse (16) eine sich hindurch erstreckende Bohrung (18) aufweist, welche mit dem Lumen (33) des Katheters (12) in Verbindung steht, wobei das Gehäuse (16) an seinem proximalen Ende (37) eine Seitenwand (60) aufweist, welche die Bohrung (18) umgreift, wobei das hämostatische Ventil (14) ferner eine geschlitzte elastische Ventiltrennwand (24) mit gegenüberliegenden Hauptflächen (61, 62) aufweist, wobei die Ventiltrennwand (24) peripher zwischen der Seitenwand (60) und einer Kappe (20) abgedichtet ist, wobei die Kappe (20) eine sich hindurch erstreckende Öffnung (22) aufweist, wobei die Bohrung (18) des Gehäuses (16) und die Öffnung (22) der Kappe (20) jeweils eine gegenüberliegende Hauptfläche (61, 62) der Ventiltrennwand (24) berühren, wobei das hämostatische Ventil (14) ferner enthält:
einen Druckring (50), der an der Kappe (20) angeordnet ist und gegen die Peripherie der Ventiltrennwand (24) stößt,
dadurch gekennzeichnet, daß
der Druckring (50) einen größeren Innendurchmesser als die Öffnung (22) und die Gehäusebohrung (18) und nicht mehr als den gleichen Durchmesser wie die Ventiltrennwand (24) aufweist, wobei der Druckring (50) die Peripherie der Trennwand (24) gegen das Gehäuse (16) drückt, was das Zentrum der Ventiltrennwand (24) dazu veranlaßt, sich zur Öffnung (22) nach außen zu biegen, und daß
die Ventiltrennwand (24) durch eine kreisförmige Scheibe ausgebildet ist, so daß die nach außen gerichtete Biegung die Abdichtung der geschlitzten Trennwand in einem Abschnitt benachbart der Hauptfläche (61) verbessert, welche die Seitenwand (60) berührt.

2. Katheter-Mantelintubator nach Anspruch 1, wobei sich das Zentrum der Ventiltrennwand nach außen biegt, so daß es in die Öffnung (22) der Kappe (20) übersteht.

3. Katheter-Mantelintubator nach einem der vorherigen Ansprüche, wobei die Ventiltrennwand (24) aus einem Elastomer ausgebildet ist.

4. Katheter-Mantelintubator nach einem der vorherigen Ansprüche, wobei die Ventiltrennwand (24) ungefähr 5 bis 20 Gewichtsprozent eines die Schmierfähigkeit erhöhenden Zusatzes enthält, der aus der Gruppe mit Wismutoxychlorid, Polytetrafluorethylen, Titandioxid, Graphit, Polyethylenwachs, Polyvinylpyrrolidon und Kombinationen hiervon ausgewählt ist.

5. Katheter-Mantelintubator nach einem der vorherigen Ansprüche, wobei die Ventiltrennwand (24) ungefähr 5 bis 15 Gewichtsprozent Graphit enthält.

6. Katheter-Mantelintubator nach einem der vorherigen Ansprüche, wobei die Ventiltrennwand (24) zwischen vier und sechs radiale Schlitze (32) definiert.

7. Katheter-Mantelintubator nach Anspruch 6, wobei sich die Schlitze (32) in einem Winkel von einer Hauptfläche zur anderen durch die Ventiltrennwand (24) erstrecken.

8. Katheter-Mantelintubator nach Anspruch 7, wobei sich die Schlitze (32) in einem Winkel zwischen 5° und 60° durch die Ventiltrennwand (24) erstrecken.

9. Katheter-Mantelintubator nach einem der vorherigen Ansprüche, wobei unterhalb der Kappe (20) durch die Gegenwart des Ringes (50) ein Freiraum vorgesehen ist, um das nach außen Biegen zu erleichtern.

## Revendications

1. Dispositif (10) d'introduction de gaine de cathéter, comportant un cathéter tubulaire (12) comprenant une extrémité distale, une extrémité proximale (32) et une lumière (33) qui traverse cette extrémité, dans lequel le cathéter porte une valve hémostatique (14) au niveau de son extrémité proximale (32), la valve hémostatique (14) comprend un boîtier (16) dont l'extrémité distale (36) est fixée à l'extrémité proximale (32) du cathéter (12), le boîtier (16) comporte un alésage( 18) qui le traverse et qui est en communication avec la lumière (33) du cathéter (12), le boîtier (16) possède une paroi d'extrémité (60) située au niveau de son extrémité proximale (37) entourant l'alésage(18), la valve hémostatique (14) comporte en outre une cloison de séparation élastique fendue (24) pourvue de surfaces principales opposées (61,62), la cloison de séparation (24) de la valve est enserrée de façon étanche, sur la périphérie, entre la paroi d'extrémité (60) et un capuchon (20), le capuchon (20) possède une ouverture (22) qui le traverse, l'alésage( 18) du boîtier (16) et l'ouverture (22) du capuchon (20) étant chacun en contact avec une surface principale opposée (61,62) de la cloison de séparation (24) de la valve, la valve hémostatique (14) comprenant en outre
une bague de compression (50) disposée sur le capuchon (20) et en butée contre la périphérie de la cloison de séparation (24) de la valve,
caractérisé en ce que
la bague de compression (50) possède un diamètre intérieur supérieur à celui de l'ouverture (22) et de l'alésage (18) du boîtier sans dépasser le diamètre de la cloison de séparation (24) de la valve, la bague de compression (50) serre la périphérie de la cloison de séparation (24) contre le boîtier (16) en provoquant le cintrage du centre de la cloison de séparation (24) de la valve vers l'extérieur en direction de l'ouverture (22), et
ladite cloison de séparation (24) de la valve est formée par un disque circulaire de sorte que ledit cintrage vers l'extérieur améliore l'étanchéité de la cloison de séparation fendue dans une partie adjacente à la surface principale (61) qui est en contact avec ladite paroi d'extrémité (60).

2. Dispositif d'introduction de gaine de cathéter selon la revendication 1, dans lequel le centre de la cloison de séparation de la valve est cintré vers l'extérieur de manière à faire saillie dans l'ouverture (22) du capuchon (20).

3. Dispositif d'introduction de gaine de cathéter selon l'une quelconque des revendications précédentes, dans lequel la cloison de séparation (24) de la valve est formée d'un élastomère.

4. Dispositif d'introduction de gaine de cathéter selon l'une quelconque des revendications précédentes, dans lequel la cloison de séparation (24) de la valve comprend environ 5 à 20 pour-cent en poids d'un additif améliorant la lubrification, choisi dans le groupe comprenant le trichlorure de bismuth, le polytétrafluoroéthylène, le dioxyde de titane, le graphite, de la cire de polyéthylène, de la polyvinylpyrolidone et des combinaisons de ces substances.

5. Dispositif d'introduction de gaine de cathéter selon l'une quelconque des revendications précédentes, dans lequel la cloison de séparation (24) de la valve comprend environ 5 à 15 pour-cent en poids de graphite.

6. Dispositif d'introduction de gaine de cathéter selon l'une quelconque des revendications précédentes, dans lequel la cloison de séparation (24) de la valve définit 4 à 6 fentes radiales (32).

7. Dispositif d'introduction de gaine de cathéter selon la revendication 6, dans lequel les fentes (32) traversent la cloison de séparation (24) de la valve, en faisant un angle défini d'une surface principale à la suivante.

8. Dispositif d'introduction de gaine de cathéter selon la revendication 7, dans lequel les fentes (32) traversent la cloison de séparation (24) de la valve sous un angle compris entre 5° et 60°.

9. Dispositif d'introduction de gaine de cathéter selon l'une quelconque des revendications précédentes, dans lequel un espace est prévu au-dessous dudit capuchon (20) en raison de la présence de ladite bague (50) de manière à faciliter ledit cintrage vers l'extérieur.
